# EUROPÄISCHE PATENTSCHRIFT

(11) **EP 2 021 068 B1**
(45) Veröffentlichungstag und Bekanntmachung des Hinweises auf die Patenterteilung: **21.02.2018**
(21) Anmeldenummer: 07718371.3
(22) Anmeldetag: 05.04.2007
(51) Int. Cl.: A61N 1/04, A61N 1/36, A61B 18/14, A61B 18/16, A61B 18/00

(54) **OBERFLÄCHENELEKTRODE**
SURFACE ELECTRODE
ÉLECTRODE DE SURFACE

(30) Priorität: 16.05.2006 AT 8422006
(43) Veröffentlichungstag der Anmeldung: 11.02.2009
(73) Patentinhaber: Medizinische Universität Wien, 1090 Wien (AT)
(72) Erfinder: MAYR, Winfried, A-2340 Mödling (AT)
(74) Vertreter: Sonn & Partner Patentanwälte
(86) Internationale Anmeldenummer: PCT/AT2007/000154
(87) Internationale Veröffentlichungsnummer: WO 2007/131248

(56) Entgegenhaltungen:
- WO-A-01/00269
- WO-A1-00/53113
- DE-A1- 2 305 220
- US-A1- 2003 004 558

## Beschreibung

Die Erfindung betrifft eine Elektrostimulations-Oberflächenelektrode zur transkutanen Elektrostimulation mit einer elektrisch leitenden Fläche zur Auflage auf die Haut, unter Anwendung eines Kontaktmediums zwischen Haut und Leitfläche, und mit zumindest einem Anschluss für eine elektrische Leitung.

Bei zahlreichen Anwendungen der funktionellen Elektrostimulation, der Elektrotherapie oder Elektrochirurgie werden plattenförmige Elektroden an der Oberfläche platziert, um elektrischen Strom in den menschlichen Körper hinein bzw. aus diesem heraus zu leiten. Derartige Elektroden sollen den Gesamtstrom möglichst gleichmäßig über die Auflagefläche verteilen und die lokale Stromdichte stets unterhalb der Schädigungsschwelle der Haut halten.

Unter elektrochirurgische Verfahren fallen beispielsweise auch Ablationstechniken, wie zur Zerstörung von Tumorgewebe.

Die Fläche derartiger bei der funktionellen Elektrostimulation, Elektrotherapie oder Elektrochirurgie verwendeten Oberflächenelektroden ist relativ groß, so dass sich starre Metallelektroden nicht besonders eignen, da sich diese nicht an die unregelmäßige Oberfläche der jeweiligen Körperregion anpassen. Aus diesem Grund existieren auch flexible Oberflächenelektroden, welche sich zumindest zu einem gewissen Grad an die Oberfläche der Hautpartie, auf welche die Oberflächenelektrode aufgelegt wird, anpassen.

Es sind verschiedene Konstruktionen von Elektroden zur Aufnahme von biologischen Signalen, wie z.B. Elektrokardiogrammen, oder zur Übertragung geringer Stromdichten, wie sie beispielsweise in der DE 196 10 246 A1, der DE 35 01 339 A1 oder der DE 41 14 677 A1 beschrieben werden, bekannt. Derartige Elektroden sind mit Oberflächenelektroden zur transkutanen Elektrostimulation, Elektrotherapie oder Elektrochirurgie gemäß der vorliegenden Beschreibung, bei welchen wesentlich höhere Stromdichten auftreten, nicht vergleichbar.

Beispielsweise beschreibt die US 3 817 252 A eine Oberflächenelektrode zur transkutanen Elektrostimulation, welche aus einem flexiblen elektrisch leitfähigen Netz, einer weiteren gitterförmigen Lage zur Verteilung des Stromes und einem mit der Haut in Kontakt stehenden Polster besteht. Durch die Schicht zur Verteilung des Stromes und dem zwischen dieser Schicht und der Hautoberfläche angeordneten elektrisch leitfähigen Polster werden so genannte "hot spots" mit unzulässigen hohen Stromdichten vermieden.

Die US 4 248 247 A beschreibt eine weitere Oberflächenelektrode, welche aus besonders flexiblem Material aufgebaut ist und über eine elektrisch leitfähige Klebeschicht auf der Hautoberfläche aufgeklebt wird. Dadurch soll die effektive Oberfläche der Elektrode vergrößert und eine gleichförmige Stromverteilung über die Fläche der Elektrode erzielt werden. Dadurch sollen lokale Stromdichte-Überhöhungen vermieden werden.

Die US 5 038 797 A beschreibt eine Elektrode zur Elektrostimulation bestehend aus einem elektrisch leitfähigen Band, aus flexiblem saugfähigen Material, welches mit Kontaktgel getränkt ist. Das Band wird über die zu stimulierende Region des Körpers, beispielsweise um eine Hand oder einen Fuß gewickelt, und mit einer Leitung zur Zuführung des elektrischen Stromes verbunden.

Die WO 01/00269 A1 beschreibt eine Vorrichtung zur Behandlung von collagenhaltigem Gewebe unter Verwendung einer Hochfrequenzelektrode, welche zur Vermeidung einer Überhitzung mit einem fließfähigen Kühlmedium durchströmt wird.

Die US 2003/0004558 A1 beschreibt eine Stimulationselektrode, welche zur Vermeidung von lokalen Stromdichtemaxima an den Rändern mit einer speziellen Silber-Silberchlorid-Schicht versehen ist.

Die WO 00/53113 A1 beschreibt eine Elektrode, welche zur Vermeidung von Stromdichteüberhöhungen an den Rändern einen zum Rand hin verringerten Widerstandsgradienten aufweist.

Schließlich zeigt die DE 2305220 A1 eine inaktive Elektrode für elektrochirurgische Eingriffe, welche ähnlich wie klassische Einweg EKG-Elektroden aufgebaut und mit einem gelförmigen Eletrolyten versehen ist.

In der Praxis kommt es jedoch immer wieder durch lokale Stromdichte-Erhöhungen zu Hautverbrennungen und Hautverätzungen. Diese lokalen Stromdichte-Erhöhungen treten insbesondere in der Randzone der Elektrode auf, was einerseits durch die dort auftretenden lokalen mechanischen Belastungen und auch durch das Austrocknen eines üblicherweise verwendeten flüssigen oder gelförmigen Kontaktmediums bedingt ist.

Insbesondere bei der funktionellen Elektrostimulation von Patienten mit Querschnittlähmung sind derartige Verletzungen an der Haut besonders gefährlich, da sie vom Patienten nicht wahrgenommen werden und deren Heilung besonders problematisch ist. Darüber hinaus werden bei der funktionellen Elektrostimulation der denervierten Muskulatur z.B. von Patienten mit schlaffer Lähmung besonders lange Impulse hoher Amplitude und somit großer Ladungsmengen verwendet, welche hohe lokale Stromdichten hervorrufen, welche zu Hautschädigungen führen können.

Die Aufgabe der vorliegenden Erfindung besteht daher in der Schaffung einer oben genannten Elektrostimulations-Oberflächenelektrode zur funktionellen Elektrostimulation, durch welche lokale Stromdichten, die über der Schädigungsschwelle der Haut liegen, wirkungsvoll vermieden werden können und somit Anwendungen auch über längere Zeiträume ohne die Gefahr von Hautschädigungen möglich werden. Die Nachteile von Elektroden gemäß dem Stand der Technik sollen vermieden oder zumindest reduziert werden. Schließlich sollen die erfindungsgemäßen Obeflächenelektroden möglichst einfach und kostengünstig herstellbar sein.

Gelöst wird die erfindungsgemäße Aufgabe durch eine oben genannte Elektrostimulations-Oberflächenelektrode, bei der am Rand der Leitfläche zumindest an der der Haut zugewandten Seite ein umlaufender Wulst aus elektrisch nicht oder schlecht leitendem Material angeordnet ist, wobei der Übergang von der Leitfläche zum Wulst stetig, beispielsweise schräg, ist, und der Rand der Leitfläche im umlaufenen Wulst eingebettet ist, der umlaufende Wulst aus elastischem Kunststoffmaterial gebildet ist, und an der, der Haut abgewandeten Seite eine elektrisch isolierende Schicht angeordnet ist. Durch den im Wesentlichen elektrisch nicht leitenden, umlaufenden Wulst am Rand der Leitfläche zumindest an der der Haut zugewandten Seite der Obeflächenelektrode wird erreicht, dass insbesondere die in der Randzone der Leitfläche auftretenden Stromdichte-Überhöhungen verhindert werden. Durch den umlaufenden Wulst und den stetigen Übergang von der Leitfläche zum Wulst wird also verhindert, dass der Rand der Leitfläche der Oberflächenelektrode in die Haut gepresst wird, wodurch die Übergangs-Impedanz reduziert und in der Folge die Stromdichte an der Randzone erhöht wird, wodurch Schädigungen der Haut hervorgerufen werden können. Dadurch, dass der umlaufende Wulst aus elektrisch nicht oder schlecht leitendem Material besteht, kann es an Stellen, wo höherer Druck auf die Haut ausgeübt wird, nicht zu einem Stromfluss mit unzulässig hoher Stromdichte kommen. Zusätzlich verhindert der umlaufende Wulst ein rasches Austrocknen eines üblicherweise verwendeten gelförmigen oder flüssigen Kontaktmediums und dadurch das lokale Auftreten von unzulässig hohen Stromdichten an derartigen Stellen. Auch wird durch den umlaufenden Wulst ein Austreten eines gelförmigen oder flüssigen Kontaktmediums wirkungsvoll verhindert, wodurch die Oberflächenelektroden auch unter der Kleidung eines Patienten auch über längere Zeitspannen angewendet werden können, ohne dass die Gefahr der Verschmutzung der Kleidungsstücke gegeben ist. Der umlaufende Wulst kann besonders einfach durch Aufkleben auf die Leitfläche der Oberflächenelektrode oder Umspritzen der Leitfläche der Oberflächenelektrode hergestellt werden, wodurch geringe Produktionskosten verbunden sind. Der stetige Übergang von der Leitfläche zum Wulst kann beispielsweise durch einen schrägen Übergang von der Leitfläche zum Wulst realisiert werden. Dadurch, dass der Rand der Leitfläche im umlaufenden Wulst eingebettet ist, wird der Rand der Leitfläche fest vom umlaufenden Wulst umgeben, so dass ein optimaler Halt desselben an der Leitfläche gegeben ist. Um zu verhindern, dass der Patient oder sonstige Personen die Leitfläche der Oberflächenelektrode von der der Haut abgewandten Seite berührt und diese dadurch elektrisiert werden, ist an der der Haut abgewandten Seite der Oberflächenelektrode eine elektrisch isolierende Schicht angeordnet. Der umlaufende Wulst ist aus elastischem Kunststoffmaterial insbesondere Silikon gebildet.

Um allfällige Druckstellen der Elektrode an der Haut zu verhindern, ist der Wulst an der der Haut zugewandten Seite vorzugsweise abgerundet ausgebildet.

Ebenso kann der Wulst an der der Haut zugewandten Seite flach ausgebildet sein.

Im Falle einer flachen Ausbildung des Wulst kann eine doppelseitige Klebefolie zur Fixierung der Oberflächenelektrode an der Haut angeordnet werden. Diese Klebefolie an der flachen, umlaufenden Fläche des Wulsts bietet einen optimalen Halt der Oberflächenelektrode an der Haut und zusätzlich eine optimale Abdichtung des Inneren der Oberflächenelektrode gegenüber der Umgebung. Durch diese Abdichtung wird ein Austrocknen des Kontaktmediums verhindert bzw. verzögert.

Die Leitfläche der Oberflächenelektrode kann durch einen elektrisch leitfähigen Kunststoff gebildet sein. Derartige elektrische leitfähige Kunststoffe können besonders flexibel hergestellt werden, resultierend in einer flexiblen Oberflächenelektrode, welche sich optimal an die Oberfläche der entsprechenden Hautregion anpasst.

Alternativ dazu kann die Leitfläche auch durch ein metallisches Geflecht gebildet sein, welches ebenfalls einen gewissen Grad an Flexibilität aufweist.

Vorteilhafterweise ist diese Isolierschicht einstückig mit dem umlaufenden Wulst gebildet. In diesem Fall wird die Leitfläche unter Bildung der Isolierschicht und dem umlaufenden Wulst in einem Herstellungsvorgang von dem elektrisch nicht oder schlecht leitendem Material umgeben. Auf diese Weise kann eine relativ kostengünstige Oberflächenelektrode hergestellt werden.

In der Isolierschicht an der der Haut abgewandten Seite der Oberflächenelektrode ist vorzugsweise der zumindest eine Anschluss für eine elektrische Leitung vorgesehen. Dabei ist der Anschluss für eine elektrische Leitung so ausgebildet, dass bei angeschlossener elektrischer Leitung eine Berührschutz gegeben ist.

Alternativ zu einem in der Isolierschicht integrierten Anschluss kann der zumindest eine Anschluss auch über eine Verbindungsleitung mit der Leitfläche der Oberflächenelektrode verbunden sein. Dadurch resultiert eine Obeflächenelektrode mit integrierten Verbindungsleitungen mit Anschlüssen bzw. Steckern, welche mit dem jeweiligen Gerät, beispielsweise Stimulationsgerät, verbunden werden können.

Die Isolierschicht ist vorzugsweise aus elastischem Kunststoffmaterial, insbesondere Silikon gebildet. Dadurch kann eine einfache Herstellung bei gleichzeitig hoher Flexibilität erzielt werden.

Das Kontaktmedium kann durch einen mit elektrisch leitfähiger Flüssigkeit getränkten Kontaktkörper gebildet sein.

Dieser Kontaktkörper ist vorzugsweise durch einen flächigen Schwamm, der zwischen Leitfläche und Haut angeordnet wird, gebildet sein. Durch den umlaufenden Wulst wird der Schwamm, insbesondere an den Randzonen, vor einem raschen Austrocknen geschützt, so dass an der Randzone keine unzulässig hohen Stromdichten resultieren.

Ebenso kann das Kontaktmedium durch ein Elektrodengel gebildet werden, welches direkt auf die der Haut zugewandten Seite der Leitfläche aufgetragen wird.

Weiters kann das Kontaktmedium durch einen Körper aus Hydrogel gebildet werden, welcher auf die der Haut zugewandten Seite der Leitfläche angeordnet wird. Ein Hydrogel ist ein wasserenthaltendes, aber wasserunlösliches Polymer, welches sich für die Anwendung bei Oberflächenelektroden besonders eignet.

Die vorliegende Erfindung wird anhand der beigefügten Zeichnungen näher erläutert.

Darin zeigen:
Fig. 1 den Teil einer Oberflächenelektrode nach dem Stand der Technik in geschnittener Darstellung;
Fig. 2 eine Ausführungsform einer Oberflächenelektrode gemäß der vorliegenden Erfindung in geschnittener Darstellung an der Randzone;
Fig. 3 eine weitere Ausführungsform einer erfindungsgemäßen Oberflächenelektrode in geschnittener Form an der Randzone;
Fig. 4 die Oberflächenelektrode gemäß Fig. 3 bei Druck auf die Randzone;
Fig. 5 eine Ansicht auf eine Oberflächenelektrode gemäß der vorliegenden Erfindung auf die der Haut abgewandten Seite;
Fig. 6 die Ansicht auf die Elektrode gemäß Fig. 5 auf die der Haut zugewandten Seite; und
Fig. 7 einen Schnitt durch die Elektrode gemäß Fig. 5 entlang der Schnittlinie VII-VII.

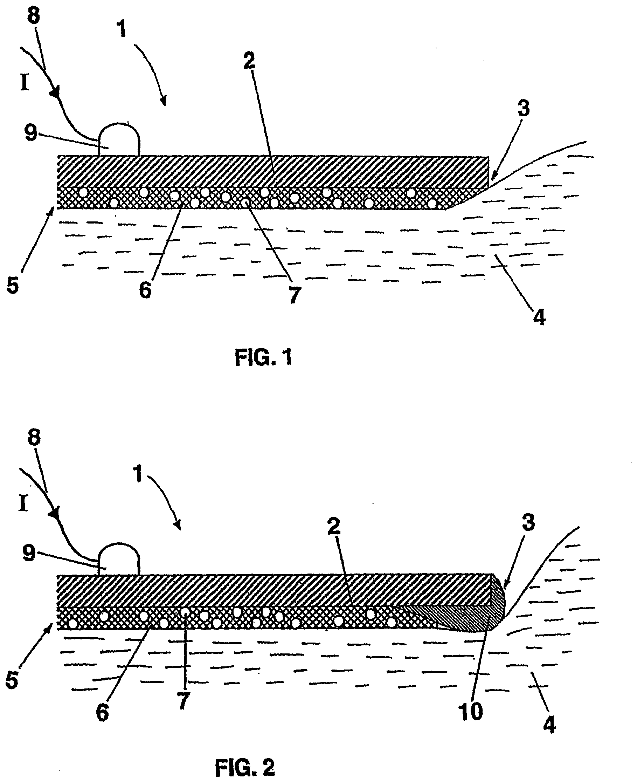

Fig. 1 zeigt eine Oberflächenelektrode 1 gemäß dem Stand der Technik in geschnittener Darstellung im Bereich des Randes 3 der elektrisch leitenden Fläche 2 bei der Auflage auf die Haut 4. Zur Reduktion des Übergangswiderstandes zwischen Leitfläche 2 und Haut 4 wird üblicherweise ein Kontaktmedium 5 beispielsweise in Form eines Kontaktkörpers 6, welcher mit elektrisch leitfähiger Flüssigkeit 7 getränkt ist, angewendet. Der Kontaktkörper 6 ist insbesondere durch einen saugfähigen Schwamm oder dgl. gebildet. Bei der elektrisch leitfähigen Flüssigkeit kann es sich beispielsweise um Kochsalzlösung oder um ein Elektrodengel handeln. Ziel der Leitfläche 2 zusammen mit dem Kontaktmedium ist es den elektrischen Strom I, der über eine elektrische Leitung 8, die mit einem Anschluss 9 verbunden ist, der Leitfläche 2 zugeführt wird, gleichmäßig zu verteilen und lokale Stromdichte-Erhöhungen zu vermeiden. Insbesondere am Rand 3 der Leitfläche 2 kommt es durch mechanische Beanspruchung zu einem höheren Gewebedruck und somit zu Veränderungen der Übergangs-Impedanz resultierend in Veränderungen der Stromdichte. Zusätzlich wird am Rand 3 der Oberflächenelektrode 1 die Flüssigkeit 7 im Kontaktkörper 6 rascher austrocknen, wodurch insbesondere am Rand 3 der Oberflächenelektrode 1 unzulässig hohe Stromdichten auftreten und Schädigungen der Haut 4 in diesen Regionen hervorrufen. Insbesondere bei der funktionellen Elektrostimulation von Patienten mit Querschnittlähmung sind derartige Verletzungen der Haut 4 unbedingt zu vermeiden, da sie von den Patienten mangels Schmerzempfindung nicht wahrgenommen werden und eine besonders große Gefahr darstellen. Die Folge sind langwierige Therapien zur Wundheilung, während welcher eine Anwendung der Oberflächenelektroden 1 zur funktionellen Elektrostimulation der Muskulatur nicht möglich ist.

Fig. 2 zeigt eine Ausführungsform einer Oberflächenelektrode 1, bei der am Rand 3 der Leitfläche 2 zumindest an der der Haut 4 zugewandten Seite ein umlaufender Wulst 10 aus elektrisch nicht oder schlecht leitendem Material angeordnet ist. Durch diesen umlaufenden Wulst 10, der beispielsweise auf die Leitfläche 2 aufgeklebt oder aufgespritzt werden kann, werden lokale Drucküberhöhungen am Rand 3 der Oberflächenelektrode 1 wirkungsvoll vermieden und somit lokale Stromdichte-Überhöhungen und in der Folge Verbrennungen der Haut 4 vermieden. Zusätzlich wirkt der umlaufende Wulst 10 als Abdichtung für das Kontaktmedium 5, welches insbesondere durch einen mit elektrisch leitfähiger Flüssigkeit 7 getränkten Kontaktkörper 6 gebildet ist. Dadurch wird auch ein Kurzschluss zwischen zwei unmittelbar nebeneinander angeordneten Oberflächenelektroden 1 wirkungsvoll verhindert. Der Übergang von der Leitfläche 2 zum Wulst 10 ist zur Vermeidung von lokalen Druckstellen stetig ausgebildet. Der Wulst 10 kann an der der Haut 4 zugewandten Seite abgerundet aber auch flach ausgebildet sein.

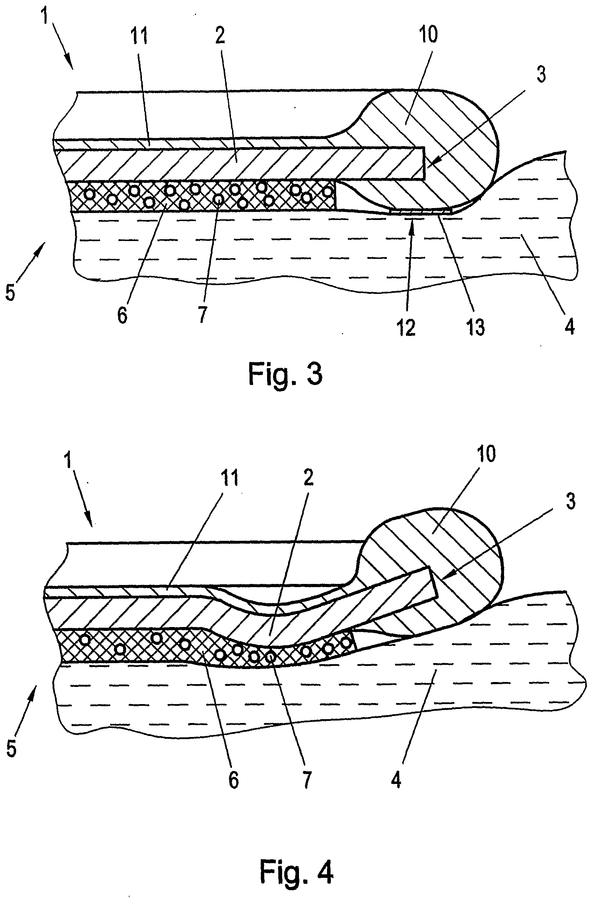

Bei der erfindungsgemäßen Oberfächenelektrode 1 gemäß Fig. 3 ist der Rand 3 der Leitfläche 2 durch den umlaufenden Wulst 10 umschlossen. Dies bietet dem Wulst 10 einen besseren Halt an der Leitfläche 2. Darüber hinaus ist an der der Haut 4 abgewandten Seite der Leitfläche 2 eine Isolierschicht 11 angeordnet und vorzugsweise zusammen mit dem Wulst 10 in einem Herstellungsvorgang an der Leitfläche 2 angeordnet. Im dargestellten Beispiel ist der Wulst 10 an der der Haut 4 zugewandten Seite 12 flach ausgebildet. Diese Fläche 12 bietet die Möglichkeit der Anwendung einer Klebefolie 13 zur Fixierung der Oberflächenelektrode 1 an der Haut 4 des Patienten.
Fig. 4 zeigt den Teil der Oberflächenelektrode 1 gemäß Fig. 3 bei lokalem Druck im Bereich des Randes 3 der Leitfläche 2. Durch den erfindungsgemäßen Wulst 10 wird verhindert, dass die Haut 4 durch die Leitfläche 2 der Oberflächenelektrode 1 mechanisch zu hoch beansprucht wird, resultierend in herabgesetzten Übergangs-Impedanzen und in der Folge hohen Stromdichten, welche zu Verletzungen führen können.

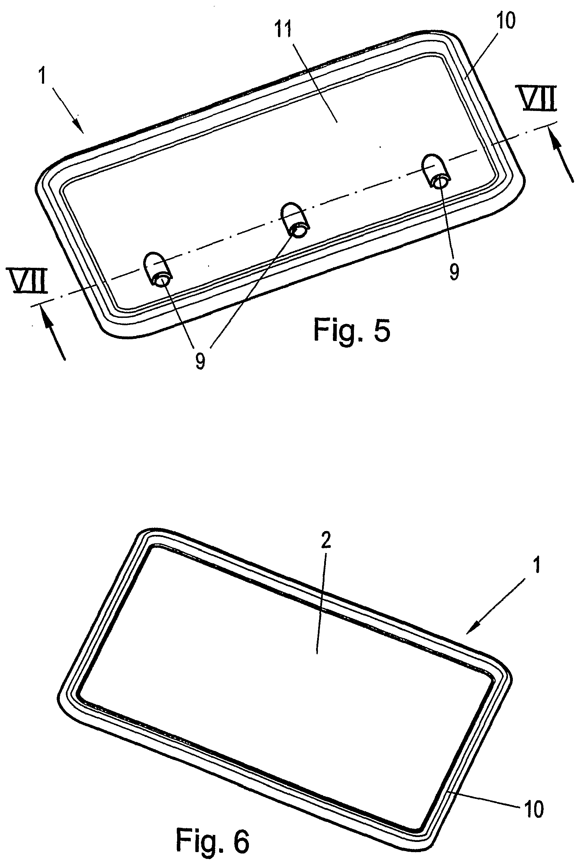

Fig. 5 zeigt eine Ansicht auf eine Ausführungsform einer Oberflächenelektrode 1, auf die der Haut 4 abgewandten Seite. Die Oberflächenelektrode 1 ist mit einem erfindungsgemäßen umlaufenden Wulst 10 und einer Isolierschicht 11 umgeben. Dadurch wird einerseits ein Schutz des Patienten vor Hautverletzungen durch zu hohe Stromdichten und ein Schutz des Patienten oder anderer Personen vor Berührung elektrisch leitender Teile erzielt. An der Isolierschicht 11 sind drei Anschlüsse 9 für elektrische Leitungen (nicht dargestellt) zur Zuführung des elektrischen Stromes angeordnet. Alternativ zu den in der Isolierschicht 11 angeordneten Anschlüssen 9 für elektrische Leitungen können die Anschlüsse 9 auch über Verbindungsleitungen mit der Leitfläche 2 verbunden werden (nicht dargestellt). In diesem Fall resultiert eine Oberflächenelektrode 1 mit integrierten Leitungen mit Anschlüssen bzw. Steckern über welche eine elektrische Verbindung zum jeweiligen Gerät, beispielsweise Stimulationsgerät hergestellt werden kann.

Fig. 6 zeigt die Ansicht auf die Oberflächenelektrode 1 gemäß Fig. 5, auf die der Haut 4 zugewandten Seite. Dabei ist die Leitfläche 2, welchen allenfalls unter Zwischenlage eines Kontaktmediums 5 auf die Haut 4 aufgelegt wird, deutlich ersichtlich. Die Leitfläche 2 ist am Rand vom umlaufenden Wulst 10 umgeben.

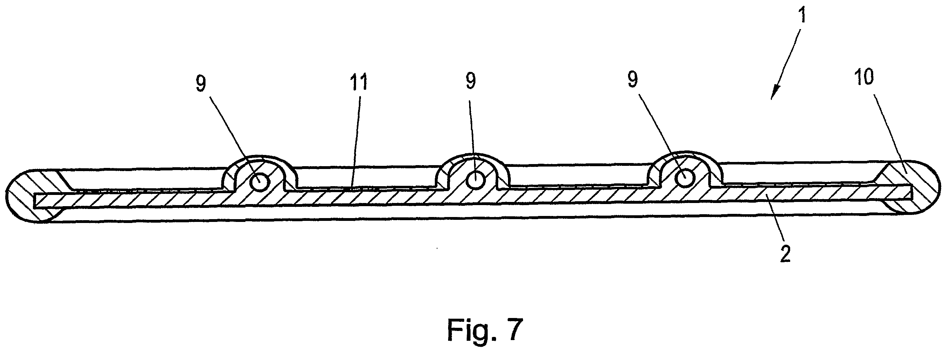

Fig. 7 zeigt ein Schnittbild durch die Oberflächenelektrode 1 gemäß Fig. 5 entlang der Schnittlinie VII-VII. Der Schnitt verläuft durch die Anschlüsse 9 für elektrische Leitungen (nicht dargestellt) zur Verbindung mit der Leitfläche 2.

Der Wulst 10 allenfalls zusammen mit der Isolierschicht 11 wird vorzugsweise aus nicht leitendem Silikon-Material hergestellt und um die Leitfläche 2, welche beispielsweise aus elektrisch leitfähigem Gummi realisiert ist, aufgespritzt. Die Größe üblicher Oberflächenelektroden 1 beträgt beispielsweise 15 x 12 cm. Durch die erfindungsgemäße Oberflächenelektrode 1 werden Hautverbrennungen und Hautverätzungen, insbesondere bei der funktionellen Elektrostimulation denervierter Muskulatur (schlaffe Lähmung) verhindert. Gleichzeitig besteht bei dieser Patientengruppe eine besondere Empfindlichkeit bezüglich Druckgeschwüren mit besonderes langwierigem Heilungsverlauf. Auch diese Druckstellenprobleme werden durch die erfindungsgemäße Oberflächenelektrode 1 reduziert bzw. verhindert.

## Patentansprüche

1. Elektrostimulations-Oberflächenelektrode (1) mit einer elektrisch leitenden Fläche (2) zur Auflage auf die Haut (4) unter Anwendung eines Kontaktmediums (5) zwischen Haut (4) und Leitfläche (2), und mit zumindest einem Anschluss (9) für eine elektrische Leitung (8), **dadurch gekennzeichnet, dass** am Rand (3) der Leitfläche (2) zumindest an der der Haut (4) zugewandten Seite ein umlaufender Wulst (10) aus elektrisch nicht oder schlecht leitendem Material angeordnet ist, wobei der Übergang von der Leitfläche (2) zum Wulst (10) stetig, beispielsweise schräg, ist, und der Rand (3) der Leitfläche (2) im umlaufenden Wulst (10) eingebettet ist, der umlaufende Wulst (10) aus elastischem Kunststoffmaterial gebildet ist, und an der, der Haut (4) abgewandten Seite eine elektrisch isolierende Schicht (11) angeordnet ist.

2. Elektrostimulations-Oberflächenelektrode (1) nach Anspruch 1, **dadurch gekennzeichnet, dass** der Wulst (10) an der der Haut (4) zugewandten Seite abgerundet ausgebildet ist.

3. Elektrostimulations-Oberflächenelektrode (1) nach Anspruch 1, **dadurch gekennzeichnet, dass** der Wulst (10) an der der Haut (4) zugewandten Seite flach ausgebildet ist.

4. Elektrostimulations-Oberflächenelektrode (1) nach Anspruch 3, **dadurch gekennzeichnet, dass** an der, der Haut (4) zugewandten Seite des umlaufenden Wulsts (10) eine Klebefolie (13) zur Fixierung an der Haut (4) vorgesehen ist.

5. Elektrostimulations-Oberflächenelektrode (1) nach einem der Ansprüche 1 bis 4, **dadurch gekennzeichnet, dass** die Leitfläche (2) durch einen elektrisch leitfähigen Kunststoff gebildet ist.

6. Elektrostimulations-Oberflächenelektrode (1) nach einem der Ansprüche 1 bis 4, **dadurch gekennzeichnet, dass** die Leitfläche (2) durch ein metallisches Geflecht gebildet ist.

7. Elektrostimulations-Oberflächenelektrode (1) nach einem der Ansprüche 1 bis 6, **dadurch gekennzeichnet, dass** die Isolierschicht (11) einstückig mit dem umlaufenden Wulst (10) gebildet ist.

8. Elektrostimulations-Oberflächenelektrode (1) nach einem der Ansprüche 1 bis 7, **dadurch gekennzeichnet, dass** der zumindest eine Anschluss (9) für eine elektrische Leitung (8) in der Isolierschicht (11) angeordnet ist.

9. Elektrostimulations-Oberflächenelektrode (1) nach einem der Ansprüche 1 bis 8, **dadurch gekennzeichnet, dass** der zumindest eine Anschluss (9) über eine Verbindungsleitung mit der Leitfläche (2) verbunden ist.

10. Elektrostimulations-Oberflächenelektrode (1) nach einem der Ansprüche 1 bis 9, **dadurch gekennzeichnet, dass** die Isolierschicht (11) aus elastischem Kunststoffmaterial, insbesondere Silikon gebildet ist.

11. Elektrostimulations-Oberflächenelektrode (1) nach einem der Ansprüche 1 bis 10, **dadurch gekennzeichnet, dass** das Kontaktmedium (5) durch einen mit elektrisch leitfähiger Flüssigkeit (7) getränkten Kontaktkörper (6) gebildet ist.

12. Elektrostimulations-Oberflächenelektrode (1) nach Anspruch 11, **dadurch gekennzeichnet, dass** der Kontaktkörper (6) durch einen flächigen Schwamm gebildet ist.

13. Elektrostimulations-Oberflächenelektrode (1) nach einem der Ansprüche 1 bis 12, **dadurch gekennzeichnet, dass** das Kontaktmedium (5) durch ein Elektrodengel gebildet ist.

14. Elektrostimulations-Oberflächenelektrode (1) nach einem der Ansprüche 1 bis 13, **dadurch gekennzeichnet, dass** das Kontaktmedium (5) durch einen Körper aus Hydrogel gebildet ist.

## Claims

1. Electrical-stimulation surface electrode (1) comprising an electrically conductive surface (2) to be applied to the skin (4) using a contact medium (5) between the skin (4) and the conductive surface (2), and comprising at least one connection (9) for an electrical line (8), **characterised in that** a peripheral bead (10), made of electrically non-conductive or poorly conductive material, is arranged on the edge (3) of the conductive surface (2), at least on the side facing the skin (4), the transition between the conductive surface (2) and the bead (10) being continuous, for example inclined, and the edge (3) of the conductive surface (2) being embedded in the peripheral bead (10), the peripheral bead (10) being made of resilient plastics material and being arranged on the side of an electrically insulating layer (11) that faces away from the skin (4).

2. Electrical-stimulation surface electrode (1) according to claim 1, **characterised in that** the side of the bead (10) that faces the skin (4) is rounded.

3. Electrical-stimulation surface electrode (1) according to claim 1, **characterised in that** the side of the bead (10) that faces the skin (4) is planar.

4. Electrical-stimulation surface electrode (1) according to claim 3, **characterised in that** an adhesive film (13) is provided on the side of the peripheral bead (10) that faces the skin (4) for securing to the skin (4).

5. Electrical-stimulation surface electrode (1) according to any of claims 1 to 4, **characterised in that** the conductive surface (2) is made of an electrically conductive plastics material.

6. Electrical-stimulation surface electrode (1) according to any of claims 1 to 4, **characterised in that** the conductive surface (2) is formed by a metal braid.

7. Electrical-stimulation surface electrode (1) according to any of claims 1 to 6, **characterised in that** the insulating layer (11) is formed integrally with the peripheral bead (10).

8. Electrical-stimulation surface electrode (1) according to any of claims 1 to 7, **characterised in that** the at least one connection (9) for an electrical line (8) is arranged in the insulating layer (11).

9. Electrical-stimulation surface electrode (1) according to any of claims 1 to 8, **characterised in that** the at least one connection (9) is connected to the conductive surface (2) by a connecting line.

10. Electrical-stimulation surface electrode (1) according to any of claims 1 to 9, **characterised in that** the insulation layer (11) is made of resilient plastics material, in particular silicone.

11. Electrical-stimulation surface electrode (1) according to any of claims 1 to 10, **characterised in that** the contact medium (5) is formed by a contact member (6) impregnated with an electrically conductive fluid (7).

12. Electrical-stimulation surface electrode (1) according to claim 11, **characterised in that** the contact body (6) is a planar sponge.

13. Electrical-stimulation surface electrode (1) according to any of claims 1 to 12, **characterised in that** the contact medium (5) is formed by an electrode gel.

14. Electrical-stimulation surface electrode (1) according to any of claims 1 to 13, **characterised in that** the contact medium (5) is formed by a hydrogel member.

## Revendications

1. Électrode d'électrostimulation de surface (1) avec une surface électro-conductrice (2) destinée à être appuyée contre la peau (4) à l'aide d'un milieu de contact (5) entre la peau (4) et la surface conductrice (2) et avec au moins un raccord (9) pour un câble électrique (8), **caractérisée en ce que**, au bord (3) de la surface conductrice (2), est disposé, au moins sur le côté orienté vers la peau (4), un bourrelet circulaire (10) constitué d'un matériau non ou faiblement électro-conducteur, la transition entre la surface conductrice (2) et le bourrelet (10) étant régulier, par exemple oblique, et le bord (3) de la surface conductrice (2) étant intégré dans le bourrelet circulaire (10), le bourrelet circulaire (10) étant constitué d'une matière plastique élastique et, sur le côté opposé à la peau (4), est disposée une couche électro-isolante (11).

2. Électrode d'électrostimulation de surface (1) selon la revendication 1, **caractérisée en ce que** le bourrelet (10) est réalisé de manière arrondie sur le côté orienté vers la peau (4).

3. Électrode d'électrostimulation de surface (1) selon la revendication 1, **caractérisée en ce que** le bourrelet (10) est réalisé de manière plane sur le côté orienté vers la peau (4).

4. Électrode d'électrostimulation de surface (1) selon la revendication 3, **caractérisée en ce que**, sur le côté, orienté vers la peau (4), du bourrelet circulaire (10), est prévu un film adhésif (13) pour la fixation sur la peau (4).

5. Électrode d'électrostimulation de surface (1) selon l'une des revendications 1 à 4, **caractérisée en ce que** la surface conductrice (2) est constituée d'une matière plastique électro-conductrice.

6. Électrode d'électrostimulation de surface (1) selon l'une des revendications 1 à 4, **caractérisée en ce que** la surface conductrice (2) est constituée d'un treillis métallique.

7. Électrode d'électrostimulation de surface (1) selon l'une des revendications 1 à 6, **caractérisée en ce que** la couche isolante (11) est formée d'une seule pièce avec le bourrelet circulaire (10).

8. Électrode d'électrostimulation de surface (1) selon l'une des revendications 1 à 7, **caractérisée en ce que** l'au moins un raccord (9) pour un câble électrique (8) est disposé dans la couche isolante (11).

9. Électrode d'électrostimulation de surface (1) selon l'une des revendications 1 à 8, **caractérisée en ce que** l'au moins un raccord (9) est relié avec la surface conductrice (2) par l'intermédiaire d'un câble de liaison.

10. Électrode d'électrostimulation de surface (1) selon l'une des revendications 1 à 9, **caractérisée en ce que** la couche isolante (11) est constituée d'une matière plastique élastique, plus particulièrement de silicone.

11. Électrode d'électrostimulation de surface (1) selon l'une des revendications 1 à 10, **caractérisée en ce que** le milieu de contact (5) est constitué d'un corps de contact (6) imprégné d'un liquide électro-conducteur (7).

12. Électrode d'électrostimulation de surface (1) selon la revendication 11, **caractérisée en ce que** le corps de contact (6) est constitué d'une éponge plate.

13. Électrode d'électrostimulation de surface (1) selon l'une des revendications 1 à 12, **caractérisée en ce que** le milieu de contact (5) est constitué d'un gel à électrodes.

14. Électrode d'électrostimulation de surface (1) selon l'une des revendications 1 à 13, **caractérisée en ce que** le milieu de contact (5) est constitué d'un corps en hydrogel.
